# FASCICULE DE BREVET EUROPEEN

(11) **EP 0 689 827 B1**
(45) Date de publication et mention de la délivrance du brevet: **31.07.1996**
(21) Numéro de dépôt: 95401268.8
(22) Date de dépôt: 31.05.1995
(51) Int. Cl.: A61K 7/09

(54) **Procédé pour la déformation permanente des matières kératiniques**
Verfahren zur dauerhaften Haarverformung
Process for permanent hair waving

(30) Priorité: 30.06.1994 FR 9408068
(43) Date de publication de la demande: 03.01.1996
(73) Titulaire: L'OREAL, F-75008 Paris (FR)
(72) Inventeur: Samain, Henri, F-91570 Bievres (FR)
(74) Mandataire: Tezier Herman, Béatrice

(56) Documents cités:
- EP-A- 0 114 414
- EP-A- 0 512 879
- DE-A- 4 117 858

## Description

La présente invention concerne un nouveau procédé de traitement des matières kératiniques, en particulier des cheveux, en vue d'obtenir une déformation permanente de ces dernières, en particulier sous la forme de cheveux permanentés, ledit procédé étant notamment utilisable dans le domaine des salons de coiffure, de beauté, de cosmétiques et analogues, professionnels.

On sait que la technique la plus usuelle pour obtenir une déformation permanente des cheveux consiste, dans un premier temps, à réaliser l'ouverture des liaisons disulfures -S-S- de la kératine (cystine) à l'aide d'une composition contenant un agent réducteur (étape de réduction) puis, après avoir de préférence rincé la chevelure ainsi traitée, à reconstituer dans un second temps lesdites liaisons disulfures en appliquant, sur les cheveux préalablement mis sous tension (bigoudis et autres), une composition oxydante (étape d'oxydation, dite aussi de fixation) de façon à donner finalement aux cheveux la forme recherchée. Cette technique permet ainsi de réaliser indifféremment soit l'ondulation des cheveux, soit leur défrisage ou leur décrêpage. La nouvelle forme imposée aux cheveux par un traitement chimique tel que ci-dessus est éminemment durable dans le temps et résiste notamment à l'action des lavages à l'eau ou des shampooings, et ceci par opposition aux simples techniques classiques de déformation temporaire, telles que de mise en plis.

Les compositions réductrices utilisables pour la mise en oeuvre de la première étape d'une opération de permanente contiennent généralement, à titre d'agents réducteurs, des sulfites, des bisulfites ou des thiols. Parmi ces derniers, on peut citer la cystéïne et ses divers dérivés, la cystéamine et ses dérivés, l'acide thiolactique, l'acide thioglycolique ainsi que ses esters, notamment le monothioglycolate de glycérol, et le thioglycérol.

Concernant les compositions oxydantes nécessaires à la mise en oeuvre de l'étape de fixation, on fait le plus souvent appel, dans la pratique, à des compositions à base d'eau oxygénée ou de bromates alcalins.

Le problème des techniques de permanentes connues à ce jour est que leur application répétée sur les cheveux induit à la longue une altération progressive de la qualité du cheveu, en particulier au niveau de sa brillance.

Un autre problème réside également dans le fait que, pour diverses raisons, il est généralement nécessaire de tamponner le pH des compositions réductrices à base de thiols par ajout de certains additifs, et en particulier de produits carbonatés comme par exemple l'acide ou le gaz carbonique, les carbonates ou les bicarbonates de métaux alcalins ou d'ammonium, les carbonates ou bicarbonates d'amines primaires, secondaires ou tertiaires, les carbonates organiques tels que notamment le carbonate de guanidine. Or, il s'avère que l'application répétée des opérations de déformations permanentes au moyen de compositions réductrices carbonatées entraine à la longue une altération progressive et marquée des propriétés cosmétiques du cheveu, en particulier au niveau de la douceur des fibres qui ont tendance à devenir de plus en plus rêches ainsi qu'au niveau de leur démêlage, les cheveux devenant de plus en plus difficiles à démêler. Cette altération est en outre particulièrement accentuée lorsque l'étape de fixation de l'opération de déformation permanente est effectuée à l'aide d'un bromate.

Le document DE-A-4 117 858 décrit un procédé pour la déformation permanente des matières kératiniques dans lequel on applique une composition de prétraitement contenant un acide carboxylique sans rincer ladite composition.

La présente invention a notamment pour but de résoudre les problèmes ci-dessus.

Plus précisément encore, la présente invention a pour but de proposer un nouveau procédé de traitement convenant à la déformation permanente des matières kératiniques et qui permet de Imiter, voire d'empêcher, la dégradation des fibres par les traitements répétés de permanentes.

Elle a également pour but de proposer un procédé tel que ci-dessus qui permette d'améliorer les propriétes cosmétiques, notamment la douceur et la facilité de démêlage, des fibres lorsque celles-ci subissent un traitement de déformation permanente au moyen d'une composition réductrice carbonatée, associée à une étape de fixation, notamment à l'aide d'un bromate.

Ainsi, à la suite d'importantes recherches menées sur la question, il a été trouvé par la Demanderesse que l'application sur les cheveux de certaines compositions acides avant l'application de la composition réductrice, pouvait permettre de remédier avec succès aux divers inconvénients qui sont liés de manière inhérente à l'application répétée sur les cheveux des opérations de permanentes, notamment des compositions réductrices, et en particulier carbonatées.

Cette découverte est à la base de la présente invention.

Or, il a été trouvé par la demanderesse que ces buts pouvaient être atteints avec succès en procédant à l'application, sur les cheveux, d'une composition acide avant l'application sur ces derniers de la composition réductrice. Cette découverte est à la base de la présente invention.

Ainsi, il est maintenant proposé selon la présente invention un nouveau procédé de traitement convenant à la déformation et/ou la mise en forme, et ceci de manière permanente, des matières kératiniques, et en particulier des cheveux, ledit procédé étant caractérisé par le fait qu'il comprend les étapes suivantes :
(i) on applique sur la matière kératinique à traiter une composition dite "composition acide" contenant au moins un acide carboxylique et/ou l'un de ses sels associés, le pH de ladite composition étant compris entre 2,5 et 7,
(ii) on rince la matière kératinique ainsi traitée,
(iii) on applique sur la matière kératinique ainsi traitée une composition réductrice contenant au moins un thiol, les moyens nécessaires à la mise sous tension mécanique de la matière kératinique étant mis en oeuvre avant, pendant, ou après ladite application de la composition réductrice ou avant ladite application de la composition acide,
(iv) puis on rince la matière kératinique ainsi traitée,
(v) on applique sur la matière kératinique ainsi rincée une composition oxydante,
(vi) et enfin on rince à nouveau la matière kératinique ainsi traitée,
la matière kératinique étant séparée des moyens de mise sous tension mentionnés à l'étape (iii) directement avant ou après le rinçage selon l'étape (vi).

D'autres caractéristiques, aspects et avantages de l'invention apparaitront plus clairement à la lecture de la description détaillée qui va suivre, ainsi que divers exemples concrets, mais nullement limitatifs, destinés à l'illustrer.

Bien que l'exposé qui suit s'articule essentiellement autour du cas particulier du traitement du cheveu, on notera ici que le procédé selon l'invention est applicable à toute matière kératinique en général, notamment cils, moustaches, poils, laine et autres.

Par acide carboxylique, on entend désigner et couvrir notamment les acides carboxyliques simples, les acides polycarboxyliques et les acides (poly)hydroxy(poly)carboxyliques, qui peuvent bien entendu être pris seuls ou en mélange.

A titre d'acides carboxyliques utilisables dans les compositions selon l'invention, on peut plus particulièrement citer les acides lactique, tartrique, acétique, glycolique et citrique.

Selon un mode particulièrement préféré de réalisation du procédé de la présente invention, l'acide mis en oeuvre est l'acide citrique.

Comme indiqué précédemment, il convient de noter que les acides carboxyliques utilisés dans l'invention peuvent être présents dans la composition acide, et ceci partiellement ou totalement, sous la forme d'un de leurs sels associés, cette présence et son ampleur dépendant en particulier du pH final imposé à ladite composition.

Le pH de la composition acide est compris de préférence entre 4 et 5,5 et peut être ajusté à l'aide d'une base choisie, seule ou en mélange, parmi la soude, la potasse, l'ammoniaque et les (poly)amines primaires, secondaires ou tertiaires.

Les (poly)amines peuvent être choisies, seules ou en mélange, parmi la monoéthanolamine, la diéthanolamine, la triéthanolamine, l'isopropanolamine, la propanediamine-1, 3 ou encore parmiles polyamines répondant à la formule : 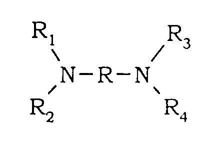 dans laquelle R est un reste propylène éventuellement substitué par un groupement hydroxyle ou un radical alkyle en C₁-C₄ et R₁, R₂, R₃ et R₄ représentent, simultanément ou indépendamment l'un de l'autre, l'hydrogène, un radical alkyle ou hydroxyalkyle en C₁-C₄.

De préférence, on met en oeuvre l'ammoniaque.

Après l'application de ladite composition acide et après avoir éventuellement laissé reposer, on rince la matière kératinique puis on applique la composition réductrice.

La concentration en acide carboxylique et/ou en leurs sels associés est généralement comprise entre 0,15 et 3 N, de préférence entre 0,3 et 1,5 N.

La composition acide peut se présenter sous forme de lotion, épaissie ou non, d'une crème, d'un gel, de shampooing, d'après-shampooing, ou de toute autre forme appropriée. Elle peut contenir des adjuvants cosmétiques connus notamment pour leur application capillaire.

Conformément à la première étape du procédé selon la présente invention (étape (i)), la composition acide est appliquée sur les cheveux à traiter, lesquels auront été de préférence préalablement mouillés. Cette application peut être réalisée après l'habituelle étape de mise sous tension des cheveux sous une forme correspondant à la forme finale désirée pour ces derniers (boucles par exemple), cette étape pouvant elle même être mise en oeuvre par tout moyen, mécanique notamment, approprié et connu en soi pour maintenir sous tension des cheveux, tels que par exemple rouleaux, bigoudis et analogues.

De préférence, on laisse ensuite reposer la chevelure sur laquelle a été appliquée la composition acide pendant une durée comprise entre 30 secondes et 30 minutes, de préférence entre 30 secondes et 10 minutes.

On rince ensuite les cheveux imprégnés de la composition acide, le rinçage étant effectué généralement à l'eau.

Dans une troisième étape indispensable du procédé selon l'invention, on applique sur les cheveux une composition réductrice, ladite composition réductrice contenant au moins un thiol.

Le thiol de la composition réductrice peut être choisi parmi les thiols connus comme agents réducteurs tels que par exemple l'acide thioglycolique, le monothioglycolate de glycérol ou de glycol, la cystéamine et ses dérivés acylés en C₁-C₄ tels que la N-acétyl cystéamine ou la N-propionyl cystéamine, la cystéine, la N-acétylcystéine, les N-mercaptoalkylamides de sucres tels que le N-(mercapto-2-éthyl)gluconamide, l'acide 2-mercaptopropionique et ses dérivés, l'acide thiolactique et ses esters tel que le monothiolactate de glycérol, l'acide thiomalique, la panthétéine, le thioglycérol, les sulfites ou les bisulfites d'un métal alcalin ou alcalino-terreux, les N-(mercaptoalkyl)ω-hydroxyalkylamides décrits dans la demande de brevet EP-A-354.835 et les N-mono- ou N,N-dialkylmercapto 4-butyramides décrits dans la demande de brevet EP-A-368.763, les aminomercaptoalkylamides décrits dans la demande de brevet EP-A-432.000, les dérivés des acides N-(mercaptoalkyl) succinamiques ou des N-(mercaptoalkyl) succiminides décrits dans la demande de brevet EP-A-465.342, les alkylaminomercaptoalkylamides décrits dans la demande de brevet EP-A-514.282, le mélange de thioglycolate d'hydroxy-2-propyle et de thioglycolate d'hydroxy-2-méthyl-1 éthyle décrit dans la demande de brevet FR-A-2.679.448.

On utilise de préférence l'acide thioglycolique, l'acide thiolactique et l'acide 2-mercaptopropionique.

Les agents réducteurs mentionnés ci-avant sont généralement présents à une concentration qui peut être comprise entre 1 et 20 % en poids par rapport au poids total de la compositon réductrice.

Le pH de la composition réductrice est généralement compris entre 7 et 9,5 et de préférence entre 7,5 et 9.

Les pH des compositions réductrices peuvent être ajustés classiquement par ajout d'agents basifiants, tels que par exemple l'ammoniaque, la monoéthanolamine, la diéthanolamine, la triéthanolamine, l'isopropanolamine, la propanediamine-1,3, l'acide ou le gaz carbonique, un carbonate ou bicarbonate de métal alcalin ou d'ammonium, un carbonate ou bicarbonate d'amines primaires, secondaires ou tertiaires, ou un carbonate organique tel que le carbonate de guanidine, tous ces composés pouvant bien entendu être pris seuls ou en mélange. Comme indiqué précédemment, l'un des grands avantages du procédé selon l'invention est qu'il convient parfaitement dans le cas des compositions réductrices carbonatées. La concentration en ion carbonate CO₃ ²⁻ dans la composition réductrice est alors généralement comprise entre 0,1 et 1,6 M.

La composition réductrice peut se présenter sous la forme d'une lotion, épaissie ou non, d'une crème, d'un gel ou de toute autre forme appropriée et peut contenir des additifs connus pour leur utilisation dans les compositions réductrices pour la déformation permanente des cheveux.

La composition réductrice peut être également du type exothermique, c'est-à-dire provoquant un certain échauffement lors de l'application sur les cheveux, ce qui apporte un agrément à la personne qui subit la permanente ou le défrisage.

La composition réductrice peut également contenir un solvant tel que par exemple de l'éthanol, du propanol, ou de l'isopropanol ou encore du glycérol à une concentration maximale de 20 % par rapport au poids total de la composition.

Lorsque les compositions sont destinées à une opération de défrisage ou de décrêpage des cheveux, la composition réductrice est de préférence sous forme d'une crème épaissie de façon à maintenir les cheveux aussi raides que possible. On réalise ces crèmes, sous forme d'émulsions "lourdes", par exemple à base de stéarate de glycéryle, de stéarate de glycol, de cires auto-émulsionnables, d'alcools gras, etc.

On peut également utiliser des liquides ou des gels contenant des agents épaississants tels que des polymères ou des copolymères carboxyvinyliques qui "collent" les cheveux et les maintiennent dans la position lisse pendant le temps de pose.

Enfin, les compositions peuvent être également sous forme dite "auto-neutralisante" ou encore "auto-régulée" et dans ce cas, les agents réducteurs utilisés selon l'invention sont associés à au moins un disulfure connu pour son utilisation dans une composition réductrice pour permanente auto-neutralisante.

Parmi de tels disulfures connus, on peut notamment mentionner l'acide dithioglycolique, le dithioglycérol, la cystamine, la N, N'-diacétyl-cystamine, la cystine, la pantéthine, et les disulfures des N-(mercapto-alkyl) ω-hydroxyalkylamides décrits dans la demande de brevet EP-A-354 835, les disulfures des N-mono ou N,N-dialkylmercapto-4 butyramides décrits dans la demande de brevet EP-A-368 763, les disulfures des aminomercapto-alkylamides décrits dans la demande de brevet EP-A-432 000, les disulfures des dérivés des acides N-(mercaptoalkyl)-succinamiques ou des N-(mercaptoalkyl)-succinimides décrits dans la demande de brevet EP-A-465 342, et les disulfures des alkylaminomercaptoalkylamides décrits dans la demande de brevet EP-A-514 282. Ces disulfures sont généralement présents dans un rapport molaire de 0,5 à 2,5, et de préférence de 1 à 2, par rapport à l'agent réducteur (voir brevet US 3 768 490).

Conformément à la troisième étape du procédé (étape (iii)), les compositions contenant le ou les agents réducteurs tels que mentionnés ci-avant sont appliqués sur les cheveux préalablement traités avec la composition acide.
Cette application peut être réalisée avant, pendant ou après l'habituelle étape de mise sous tension des cheveux.

Avant de procéder à l'étape suivante de rinçage, il convient, de manière classique, de laisser reposer pendant quelques minutes, généralement entre 2 et 40 minutes, de préférence entre 5 et 30 minutes, la chevelure sur laquelle a été appliquée la composition réductrice, et ceci de façon à bien laisser le temps au réducteur d'agir correctement sur les cheveux. Cette phase d'attente est effectuée généralement en laissant reposer à l'air libre (température ambiante) la chevelure traitée. Pendant cette phase d'attente, on prend soin que les cheveux ne sèchent pas complètement et restent humides jusqu'au moment de la mise en oeuvre de l'étape suivante (à cet effet, utilisation possible de bonnets, de gels de protection par exemple).

Dans la quatrième étape du procédé (étape (iv)), les cheveux imprégnés de la composition réductrice sont donc ensuite rincés soigneusement, généralement à l'eau.

Puis, dans une cinquième étape (étape (v)), on applique sur les cheveux ainsi rincés une composition oxydante dans le but de fixer la nouvelle forme imposée aux cheveux.

La composition oxydante contient un agent oxydant qui peut être choisi parmi l'eau oxygénée, un bromate alcalin, un persel ou un polythionate. Comme indiqué précédemment, l'un des grands avantages du procédé selon l'invention est qu'il convient parfaitement dans le cas des compositions oxydantes à base de bromates. La concentration en bromates dans la composition oxydante est généralement comprise entre 0,1 et 2 M.

Le pH de la composition oxydante est généralement compris entre 2 et 10.

La composition oxydante peut contenir des additifs cosmétiques bien connus pour ce type de composition.

Comme dans le cas de l'application de la composition réductrice, la chevelure sur laquelle a été appliquée la composition oxydante est ensuite, de manière classique, laissée dans une phase de repos ou d'attente qui dure quelques minutes, généralement entre 3 et 30 minutes, de préférence entre 5 et 15 minutes.

Le véhicule des compositions utilisées selon l'invention (composition acide, composition réductrice, composition oxydante) est de préférence l'eau ou une solution hydroalcoolique d'un alcool inférieur tel que l'éthanol, l'isopropanol ou le butanol.

Enfin, dans la dernière étape du procédé selon l'invention (étape (vi)), les cheveux imprégnés de la composition oxydante sont rincés soigneusement, généralement à l'eau. On sépare, directement avant ou après le rinçage, la matière kératinique des moyens de tensions mentionnés à l'étape (iii).

On obtient finalement une chevelure présentant de bonnes propriétés cosmétiques : les cheveux sont plus brillants, plus doux et plus faciles à démêler.

Des exemples concrets illustrant l'invention vont maintenant être donnés.

### Exemple 1

On a utilisé des mèches contenant 8 g de cheveux japonais. Pour les sensibiliser, on a effectué une décoloration à l'aide d'une composition contenant de l'eau oxygénée à 20 volumes à laquelle on ajoute poids pour poids une lotion alcaline contenant 2 % d'ammoniaque pure. On laisse agir la composition pendant 30 minutes. On obtient ainsi des mèches décolorées qui sont utilisées pour les essais suivants.

On a réalisé quatre procédés de permanente : trois procédés (n°1, 2 et 3) avec une lotion acide appliquée à des phases différentes du procédé et un procédé (n°4) sans lotion acide.

Pour ces procédés, on a utilisé les compositions suivantes :

### Lotion acide n° 1 :

| | |
|---|---|
| - acide citrique | 10 g |
| - ammoniaque à 20 % | qs pH 4, 5 |
| - chlorure d'oléocétyl diméthyl hydroxyéthyl ammonium | 0, 4 g MA |
| - eau déminéralisée | qs 100 g |

### Composition réductrice n° 1 :

| | |
|---|---|
| - acide thioglycolique | 8 g |
| - carbonate d'ammonium | 6 g |
| - ammoniaque à 20 % | qs pH 8, 1 |
| - eau déminéralisée | qsp 100 g |

### Composition oxydante n° 1 :

| | |
|---|---|
| - bromate de sodium | 7 g |
| - acide phosphorique | qs pH 8 |
| - eau déminéralisée | qsp 100 g |

On a réalisé les procédés de permanentes suivants :

### Procédé n° 1 (invention) :

On a appliqué sur des mèches humides la lotion acide n°1 puis, après 5 minutes de pose, on a rincé les cheveux. On a enroulé les mèches ainsi traitées sur 5 bigoudis de 9 mm de diamètre puis on a appliqué sur les cheveux ainsi enroulés la composition réductrice n°1 ; on a laissé agir pendant 15 minutes puis on a rincé. On a ensuite appliqué la composition oxydante n°1, on a laissé agir pendant 15 minutes puis on a rincé les cheveux. Enfin, on a déroulé les cheveux des bigoudis puis on a séché les cheveux.

### Procédé n° 2 (comparatif)

On a effectué un procédé analogue au procédé n° 1, à la seule différence que l'application de la lotion acide n°1 est réalisée après l'application de la composition réductrice n°1.

### Procédé n° 3 (comparatif)

On a effectué un procédé analogue au procédé n° 1, à la seule différence que l'application de la lotion acide n°1 est réalisée après l'application de la composition oxydante n°1.

### Procédé n° 4 (comparatif)

On a effectué un procédé analogue au procédé n°1 mais sans appliquer de lotion acide.

On a évalué, par un pannel de 12 juges, la douceur et la facilité de démêlage des mêches traitées par les différents procédés.

La douceur a été notée de 0 à 5 :
- 0: cheveux très rêches
- 1: cheveux rêches
- 2: cheveux assez rêches
- 3: cheveux assez doux
- 4: cheveux doux
- 5: cheveux très doux

La facilité de démêlage a été notée de 0 à 5 :
- 0: démêlage impossible (le peigne ne passe pas)
- 1: démêlage très difficile (une vingtaine de coups de peigne sont nécessaires pour démêler 8 g de cheveux)
- 2: démêlage difficile (une dizaine de coups de peigne sont nécessaires pour démêler 8 g de cheveux)
- 3: démêlage assez facile (4 à 5 coups de peigne sont nécessaires pour démêler 8 g de cheveux
- 4: démêlage facile (un seul coup de peigne est nécessaire)
- 5: démêlage très facile (dès le premier coup de peigne, les cheveux n'accrochent pas)

Les moyennes des notes données ont été les suivantes :

| **Procédé n°** | **Douceur** | **Démêlage** |
|---|---|---|
| 1 (invention) | 3, 1 | 4 |
| 2 | 2, 5 | 2, 8 |
| 3 | 1, 1 | 1 |
| 4 | 1, 1 | 1, 25 |

On a donc constaté qu'en appliquant la lotion acide avant l'étape de réduction, on obtient les meilleurs résultats de douceur et de démêlage.

### Exemple 2

On a effectué 6 shampooings (lavage, rinçage et séchage) sur les cheveux traités selon le procédé n° 1 de l'exemple 1 et on a répété le procédé de traitement n° 1 de l'exemple 1.

De même, on a effectué 6 shampooings (lavage, rinçage et séchage) sur les cheveux traités selon le procédé n° 2 de l'exemple 1 et on a répété le procédé de traitement n° 2 de l'exemple 1.

On a évalué, comme à l'exemple 1, la douceur et la facilité de démêlage des cheveux ainsi traités ; on a obtenu les résultats suivants :

| **Procédé n°** | **Douceur** | **Démêlage** |
|---|---|---|
| 1 (invention) | 3, 0 | 3, 8 |
| 2 | 1, 1 | 1, 5 |

On a ainsi constaté que l'écart de notation de douceur et de démêlage s'est accentué en faveur des cheveux traités selon l'invention.

### Exemple 3 : comparaison du pH de la lotion acide

On a réalisé le procédé n° 1 en utilisant deux lotions acides différentes : on a utilisé la lotion acide n°1 (procédé n°1) et la lotion acide n° 2 (procédé n°1') qui avait les caractéristiques suivantes :

### Lotion acide n° 2 :

| | |
|---|---|
| - acide citrique | 10 g |
| - ammoniaque à 20 % | qs pH 9 |
| - chlorure d'oléocétyl diméthyl hydroxyéthyl ammonium | 0, 4 g MA |
| - eau déminéralisée | qsp 100 g |

On a effectué les procédés n°1 et n°1' sur la chevelure de trois femmes japonaises, le procédé n°1 étant appliqué du coté gauche, le procédé n°1' étant appliqué du coté droit .

On a évalué, comme à l'exemple 1, la douceur des cheveux ainsi traités à l'état humide. On a évalué également la douceur et la brillance des cheveux une fois secs ; on a obtenu les résultats suivants :

| **Procédé n°** | **Douceur** cheveux humides | **Douceur** cheveux secs | **Brillance** cheveux secs |
|---|---|---|---|
| 1 (invention) | 3 | 3 | 3, 2 |
| 1' | 2, 2 | 1 | 1, 5 |

On a ainsi constaté que les cheveux traités avec une lotion acide selon l'invention (pH 4,5) avaient une douceur et une brillance supérieures à celles des cheveux traités avec une lotion acide (n°1') ayant un pH différent (pH 9).

### Exemple 4

On a utilisé des mèches contenant 8 g de cheveux européens. Pour les sensibiliser, on a effectué deux décolorations à l'aide d'une composition contenant de l'eau oxygénée à 20 volumes à laquelle on ajoute poids pour poids une lotion alcaline contenant 2 % d'ammoniaque pure. On laisse agir la composition pendant 30 minutes. On obtient ainsi des mèches décolorées qui sont utilisées pour l'exemple de réalisation suivant.

On a réalisé le procédé n°1 mais en utilisant une composition oxydante qui avait les caractéristiques suivantes :

### Composition oxydante n°2 :

| | |
|---|---|
| - eau oxygénée | qs 8 volumes |
| - acide phosphorique | qs pH 3 |
| - eau déminéralisée | qsp 100 g |

On a constaté que les cheveux avant séchage sont doux et faciles à démêler. Une fois secs, les cheveux sont doux et brillants.

## Revendications

1. Procédé de traitement pour la déformation permanente des matières kératiniques, caractérisé par le fait qu'il comprend les étapes suivantes :
(i) on applique sur la matière kératinique une composition contenant au moins un acide carboxylique et/ou l'un de ses sels associés (composition acide), le pH de la dite composition étant compris entre 2,5 et 7,
(ii) on rince la matière kératinique ainsi traitée,
(iii) puis on applique sur la matière kératinique ainsi traitée une composition réductrice contenant au moins un thiol, les moyens nécessaires à la mise sous tension mécanique de la matière kératinique étant mis en oeuvre avant, pendant ou après ladite application de la composition réductrice ou avant ladite application de la composition acide,
(iv) puis on rince la matière kératinique ainsi traitée,
(v) on applique sur la matière kératinique ainsi rincée une composition oxydante,
(vi) et enfin on rince à nouveau la matière kératinique ainsi traitée,
la matière kératinique étant séparée des moyens de mise sous tension mentionnés à l'étape (iii) directement avant ou après le rinçage selon l'étape (vi).

2. Procédé selon la revendication 1, caractérisé par le fait que ledit acide de la composition appliquée selon l'étape (i) est choisi, seul ou en mélange, parmi les acides carboxyliques simples, les acides polycarboxyliques, les acides (poly)hydroxy(poly)carboxyliques.

3. Procédé selon la revendication 2, caractérisé par le fait que ledit acide carboxylique est choisi, seul ou en mélange, parmi les acides lactique, tartrique, acétique, glycolique et citrique.

4. Procédé selon la revendication 3, caractérisé par le fait que ledit acide carboxylique est l'acide citrique.

5. Procédé selon l'une des revendications précédentes, caractérisé par le fait que le pH de ladite composition acide est ajusté à l'aide d'une base choisie, seule ou en mélange, parmi la soude, la potasse, l'ammoniaque et les (poly)amines primaires, secondaires ou tertiaires.

6. Procédé selon la revendication 5, caractérisé par le fait que lesdites polyamines répondent à la formule générale : 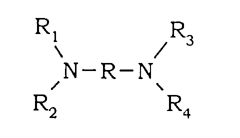 dans laquelle R est un reste propylène éventuellement substitué par un groupement hydroxyle ou un radical akyle en C₁-C₄ et R₁, R₂, R₃ et R₄ représentent, simultanément ou indépendamment l'un de l'autre, l'hydrogène, un radical alkyle ou hydroxyalkyle en C₁-C₄.

7. Procédé selon l'une des revendications 5 ou 6, caractérisée en ce que lesdites (poly)amines sont choisies, seules ou en mélange, parmi la monoéthanolamine, la diéthanolamine, la triéthanolamine, l'isopropanolamine et la propanediamine-1,3.

8. Procédé selon la revendication 5, caractérisé par le fait que la base est l'ammoniaque.

9. Procédé selon l'une quelconque des revendications précédentes, caractérisée par le fait que la concentration en acide carboxylique et/ou en leurs sels associés est comprise entre 0,15 et 3 N, de préférence entre 0,3 et 1, 5 N.

10. Procédé selon l'une quelconque des revendications précédente, caractérisé par le fait que le pH de la composition acide est compris entre 4 et 5,5.

11. Procédé selon l'une quelconque des revendications précédentes, caractérisé par le fait que, avant de procéder au rinçage (étape (ii)), on laisse reposer la matière kératinique issue de l'étape (i).

12. Procédé selon la revendication 11, caractérisé par le fait que la phase de repos dure entre 30 secondes et 30 minutes, de préférence entre 30 secondes et 10 minutes.

13. Procédé selon l'une quelconque des revendications précédentes, caractérisé par le fait que ledit thiol de la composition réductrice est choisi parmi l'acide thioglycolique, l'acide thiolactique, l'acide 2-mercaptopropionique.

14. Procédé selon l'une des revendications précédentes, caractérisé par le fait que ledit thiol est présent à une concentration comprise entre 1 et 20 % en poids par rapport au poids total de la composition réductrice.

15. Procédé selon l'une quelconque des revendications précédentes, caractérisé par le fait que le pH de la composition réductrice est compris entre 7 et 9, 5, de préférence entre 7,5 et 8, 6.

16. Procédé selon l'une quelconque des revendications précédentes, caractérisé par le fait que la composition réductrice est une composition réductrice carbonatée.

17. Procédé selon la revendication 16, caractérisé par le fait que la composition réductrice est carbonatée à l'aide d' un additif choisi parmi l'acide ou le gaz carbonique, les carbonates ou bicarbonates de métaux alcalins ou d'ammonium,les carbonates ou bicarbonates d'amines primaires, secondaires ou tertiaire, les carbonates organiques tels que le carbonate de guanidine.

18. Procédé selon les revendications 16 ou 17, caractérisé par le fait que la teneur en ion carbonate CO₃ ²⁻ dans la composition réductrice est comprise entre 0,1 et 1,6 M.

19. Procédé selon l'une quelconque des revendications précédentes, caractérisé par le fait que la composition réductrice contient en outre au moins un disulfure, la composition étant de type auto-neutralisante.

20. Procédé selon l'une quelconque des revendications précédentes, caractérisé par le fait que la composition oxydante contient un agent oxydant choisi parmi l'eau oxygénée, un bromate alcalin, un persel ou un polythionate.

21. Procédé selon la revendication 20, caractérisé par le fait que l'agent oxydant est un bromate alcalin.

22. Procédé selon l'une quelconque des revendications précédentes, caractérisé par le fait que ladite matière kératinique consiste en des cheveux.

## Claims

1. Treatment process for the permanent reshaping of keratinous material, characterized in that it comprises the following steps:
(i) a composition containing at least one carboxylic acid and/or one of the associated salts thereof (acidic composition) is applied to the keratinous material, the pH of the said composition being between 2.5 and 7,
(ii) the keratinous material thus treated is rinsed,
(iii) a reducing composition containing at least one thiol is then applied to the keratinous material thus treated, the means necessary for placing the keratinous material under mechanical tension being implemented before, during or after the said application of the reducing composition or before the said application of the acidic composition,
(iv) the keratinous material thus treated is then rinsed,
(v) an oxidizing composition is applied to the keratinous material thus rinsed,
(vi) and, finally, the keratinous material thus treated is rinsed again,
the keratinous material being separated from the means for placing under tension mentioned in step (iii) directly before or after the rinsing operation in step (vi).

2. Process according to Claim 1, characterized in that the said acid of the composition applied in step (i) is chosen, alone or as a mixture, from simple carboxylic acids, polycarboxylic acids and (poly)hydroxy(poly)carboxylic acids.

3. Process according to Claim 2, characterized in that the said carboxylic acid is chosen, alone or as a mixture, from lactic acid, tartaric acid, acetic acid, glycolic acid and citric acid.

4. Process according to Claim 3, characterized in that the said carboxylic acid is citric acid.

5. Process according to one of the preceding claims, characterized in that the pH of the said acidic composition is adjusted using a base chosen, alone or as a mixture, from sodium hydroxide, potassium hydroxide, aqueous ammonia and primary, secondary or tertiary (poly)amines.

6. Process according to Claim 5, characterized in that the said polyamines correspond to the general formula: 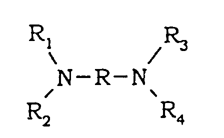 in which R is a
propylene residue optionally substituted with a hydroxyl group or a C₁-C₄ alkyl radical and R₁, R₂, R₃ and R₄ represent, simultaneously or independently of each other, hydrogen or a C₁-C₄ alkyl or hydroxyalkyl radical.

7. Process according to either of Claims 5 and 6, characterized in that the said (poly)amines are chosen, alone or as a mixture, from monoethanolamine, diethanolamine, triethanolamine, isopropanolamine and 1,3-propanediamine.

8. Process according to Claim 5, characterized in that the base is aqueous ammonia.

9. Process according to any one of the preceding claims, characterized in that the concentration of carboxylic acid and/or of associated salts thereof is between 0.15 and 3 N, preferably between 0.3 and 1.5 N.

10. Process according to any one of the preceding claims, characterized in that the pH of the acidic composition is between 4 and 5.5.

11. Process according to any one of the preceding claims, characterized in that, before performing the rinsing operation (step (ii)), the keratinous material obtained from step (i) is left to stand.

12. Process according to Claim 11, characterized in that the standing phase lasts for between 30 seconds and 30 minutes, preferably between 30 seconds and 10 minutes.

13. Process according to any one of the preceding claims, characterized in that the said thiol of the reducing composition is chosen from thioglycolic acid, thiolactic acid and 2-mercaptopropionic acid.

14. Process according to one of the preceding claims, characterized in that the said thiol is present at a concentration between 1 and 20 % by weight relative to the total weight of the reducing composition.

15. Process according to any one of the preceding claims, characterized in that the pH of the reducing composition is between 7 and 9.5, preferably between 7.5 and 8.6.

16. Process according to any one of the preceding claims, characterized in that the reducing composition is a carbonate-based reducing composition.

17. Process according to Claim 16, characterized in that the reducing composition is carbonated using an additive chosen from carbonic acid or carbon dioxide, ammonium or alkali metal carbonates or bicarbonates, primary, secondary or tertiary amine carbonates or bicarbonates, and organic carbonates such as guanidine carbonate.

18. Process according to Claims 16 or 17, characterized in that the content of carbonate ion CO₃²⁻ in the reducing composition is between 0.1 and 1.6 M.

19. Process according to any one of the preceding claims, characterized in that the reducing composition additionally contains at least one disulphide, the composition being of self-neutralizing type.

20. Process according to any one of the preceding claims, characterized in that the oxidizing composition contains an oxidizing agent chosen from aqueous hydrogen peroxide solution, an alkali metal bromate, a persalt or a polythionate.

21. Process according to Claim 20, characterized in that the oxidizing agent is an alkali metal bromate.

22. Process according to any one of the preceding claims, characterized in that the said keratinous material consists of hair.

## Patentansprüche

1. Behandlungsverfahren für die dauerhafte Verformung keratinischer Materialien, das durch folgende Schritte gekennzeichnet ist:
(i) Auftragen auf das keratinische Material einer Zusammensetzung, die mindestens eine Carbonsäure und/oder eines ihrer Salze enthält (saure Zusammensetzung), wobei der pH-Wert der Zusammensetzung im Bereich von 2,5 bis 7 liegt,
(ii) Spülen des so behandelten keratinischen Materials,
(iii) anschließend Auftragen einer reduzierenden Zusammensetzung, die mindestens ein Thiol enthält auf das so behandelte keratinische Material, wobei die Mittel, die erforderlich sind, um das keratinische Material unter mechanische Spannung zu setzen, vor, während oder nach dem Auftragen der reduzierenden Zusammensetzung oder vor dem Auftragen der sauren Zusammensetzung angewendet werden,
(iv) anschließend Spülen des so behandelten keratinischen Materials,
(v) Auftragen einer oxidierenden Zusammensetzung auf das so gespülte keratinische Material
(vi) und schließlich erneutes Spülen des so behandelten keratinischen Materials,
wobei das keratinische Material unmittelbar vor oder nach dem Spülen gemäß Schritt (vi) von den in Schritt (iii)-genannten Mitteln, mit denen es unter Spannung gesetzt wird, getrennt wird.

2. Verfahren nach Anspruch 1, dadurch gekennzeichnet, daß die Säure der gemäß Schritt (i) aufgetragenen Zusammensetzung unter Monocarbonsäuren, Polycarbonsäuren und (Poly)hydroxy(polycarbonsäuren) ausgewählt wird, die allein oder im Gemisch verwendet werden.

3. Verfahren nach Anspruch 2, dadurch gekennzeichnet, daß die Carbonsäure unter Milchsäure, Weinsäure, Essigsäure, Glykolsäure und Citronensäure ausgewählt wird, die allein oder im Gemisch verwendet werden.

4. Verfahren nach Anspruch 3, dadurch gekennzeichnet, daß es sich bei der Carbonsäure um Citronensäure handelt.

5. Verfahren nach einem der vorhergehenden Ansprüche, dadurch gekennzeichnet, daß der pH-Wert der sauren Zusammensetzung mit einer Base eingestellt wird, die unter Natriumhydroxid, Kaliumhydroxid, Ammoniak und primären, sekundären und tertiären (Poly)aminen ausgewählt wird, die allein oder im Gemisch verwendet werden.

6. Verfahren nach Anspruch 5, dadurch gekennzeichnet, daß die Polyamine die allgemeine Formel 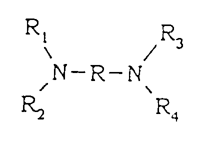 aufweisen, in der R ein ggf. mit einer Hydroxygruppe oder C₁₋₄-Alkyl substituierter Propylenrest ist und R₁, R₂, R₃ und R₄ gleichzeitig oder unabhängig voneinander Wasserstoff, C₁₋₄-Alkyl oder C₁₋₄-Hydroxyalkyl darstellen.

7. Verfahren nach einem der Ansprüche 5 und 6, dadurch gekennzeichnet, daß die (Poly)amine unter Monoethanolamin, Diethanolamin, Triethanolamin, Isopropanolamin und 1,3-Diaminopropan ausgewählt werden, die allein oder im Gemisch verwendet werden.

8. Verfahren nach Anspruch 5, dadurch gekennzeichnet, daß es sich bei der Base um Ammoniak handelt.

9. Verfahren nach einem der vorhergehenden Ansprüche, dadurch gekennzeichnet, daß die Konzentration der Carbonsäure und/oder der Salze dieser Carbonsäure im Bereich von 0,15 bis 3 N, vorzugsweise 0,3 bis 1,5 N, liegt.

10. Verfahren nach einem der vorhergehenden Ansprüche, dadurch gekennzeichnet, daß der pH-Wert der sauren Zusammensetzung 4 bis 5,5 beträgt.

11. Verfahren nach einem der vorhergehenden Ansprüche, dadurch gekennzeichnet, daß man das aus Schritt (i) hervorgegangene keratinische Material ruhen läßt, bevor gemäß Schritt (ii) gespült wird.

12. Verfahren nach Anspruch 11, dadurch gekennzeichnet, daß die Phase, in der man das keratinische Material ruhen läßt, 30 s bis 30 min, vorzugsweise 30 s bis 10 min, dauert.

13. Verfahren nach einem der vorhergehenden Ansprüche, dadurch gekennzeichnet, daß das Thiol der reduzierenden Zusammensetzung unter Thioglykolsäure, Thiomilchsäure und 2-Mercaptopropionsäure ausgewählt wird.

14. Verfahren nach einem der vorhergehenden Ansprüche, dadurch gekennzeichnet, daß das Thiol in einer Konzentration von 1 bis 20 Gew.-%, bezogen auf das Gesamtgewicht der reduzierenden Zusammensetzung, enthalten ist.

15. Verfahren nach einem der vorhergehenden Ansprüche, dadurch gekennzeichnet, daß der pH-Wert der reduzierenden Zusammensetzung 7 bis 9,5, vorzugsweise 7,5 bis 8,6, beträgt.

16. Verfahren nach einem der vorhergehenden Ansprüche, dadurch gekennzeichnet, daß die reduzierende Zusammensetzung eine carbonathaltige reduzierende Zusammensetzung ist.

17. Verfahren nach Anspruch 16, dadurch gekennzeichnet, daß die reduzierende Zusammensetzung carbonathaltig ist aufgrund eines Zusatzes, der unter Kohlensäure, Kohlendioxid, Alkalimetallcarbonaten und -hydrogencarbonaten, Ammoniumcarbonat und Ammoniumhydrogencarbonat, Carbonaten und Hydrogencarbonaten primärer, sekundärer oder tertiärer Amine und organischen Carbonaten, wie z.B. Guanidiniumcarbonat, ausgewählt wird.

18. Verfahren nach Anspruch 16 oder 17, dadurch gekennzeichnet, daß der Gehalt der reduzierenden Zusammensetzung an Carbonationen CO₃²⁻ 0,1 bis 1,6 M beträgt.

19. Verfahren nach einem der vorhergehenden Ansprüche, dadurch gekennzeichnet, daß die reduzierende Zusammensetzung ferner mindestens ein Disulfid enthält, wobei die Zusammensetzung eine selbstneutralisierende Zusammensetzung ist.

20. Verfahren nach einem der vorhergehenden Ansprüche, dadurch gekennzeichnet, daß die oxidierende Zusammensetzung ein Oxidationsmittel enthält, das unter Wasserstoffperoxid, Alkalibromaten, Persalzen und Polythionaten ausgewählt wird.

21. Verfahren nach Anspruch 20, dadurch gekennzeichnet, daß es sich bei dem Oxidationsmittel um ein Alkalibromat handelt.

22. Verfahren nach einem der vorhergehenden Ansprüche, dadurch gekennzeichnet, daß das keratinische Material aus Haaren besteht.
